(19) Europäisches Patentamt
European Patent Office
Office européen des brevets

(11) Publication number: **0 134 005**
**B1**

# (12) EUROPEAN PATENT SPECIFICATION

(45) Date of publication of patent specification: 17.11.88

(51) Int. Cl.$^4$: **C 07 C 69/54**, C 07 C 67/10

(21) Application number: 84109315.6

(22) Date of filing: 06.08.84

## (54) Process for preparing triarylmethyl methacrylate.

(30) Priority: 17.08.83 JP 150038/83
11.10.83 JP 188326/83

(43) Date of publication of application:
13.03.85 Bulletin 85/11

(45) Publication of the grant of the patent:
17.11.88 Bulletin 88/46

(84) Designated Contracting States:
BE CH DE FR GB IT LI NL

(56) References cited:

CHEMICAL ABSTRACTS, vol. 102, no. 16, 22nd April 1985, p. 598, no. 140874n, Columbus, Ohio (US)

CHEMICAL ABSTRACTS, vol. 98, no. 7, 14th February 1983, p. 607, no. 53171m, Columbus, Ohio (US)

(73) Proprietor: DAICEL CHEMICAL INDUSTRIES, LTD.
No. 1-Banchi, Teppo-cho
Sakai-shi Osaka-fu 590 (JP)

(72) Inventor: Yuki, Yoichi
1903-3, Okihama Aboshi-ku
Himeji-shi Hyogo (JP)
Inventor: Noyori, Ryoji
135-417, Umemori-shinden Nisshin-cho
Aichi-gun Aichi (JP)
Inventor: Hayashi, Masahiko
13-34, Sumiike-cho Nakagawa-ku
Nagoya-shi Aichi (JP)

(74) Representative: Patentanwälte Grünecker, Dr. Kinkeldey, Dr. Stockmair, Dr. Schumann, Jakob, Dr. Bezold, Meister, Hilgers, Dr. Meyer-Plath
Maximilianstrasse 58
D-8000 München 22 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 134 005 B1

## Description

The present invention relates to a process for producing a triarylmethyl methacrylate of formula I

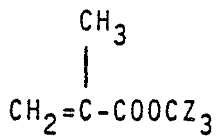

(I)

wherein Z is an aryl group which may be substituted.

The product obtained in the process is useful as a starting monomer to produce a polymer having optical resolving power. The use of the product is disclosed in Japanese patent publication No. 106907/1981.

Heretofore, triphenylmethylation reaction has been utilized in most cases for protecting carboxyl groups of synthetic intermediates, so that it has been applied to various acids. Since formed triphenylmethyl esters merely served as intermediates, there have been scarcely any problems in the separation and purification of the esters. In the production of the above-mentioned monomer, wherein the acid component is restricted to methacrylic acid, however, it is industrially important to efficiently isolate highly pure esters while preventing them from being polymerized and hydrolyzed.

It is known that triphenylmethyl esters of carboxylic acids are liable to undergo rapid hydrolysis and hence it is impossible to obtain these esters by dehydration reaction between an acid and an alcohol. Heretofore, triphenylmethyl esters have been prepared by reacting triphenylmethyl chloride or bromide with a metal salt of a carboxylic acid in a non-polar solvent. Particularly when esterifying an acid liable to undergo a side reaction, for example, methacrylic acid liable to undergo polymerization, the reaction had to be carried out by using an expensive silver salt as represented by the following equation:

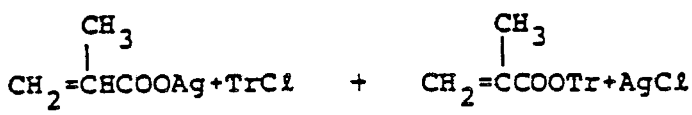

wherein Tr represents a triphenylmethyl group.

This reaction is described in the literature (N. A. Adrova and L. K. Prokhorova, Vysokomolekulyarnye Soedineniya *3*, 1509 (1961)). This method using an equivalent of silver causes problems from the viewpoints of economy and natural resources.

As a process for producing triphenylmethyl methacrylate using no silver salt, only a process using trialkylsilyl esters is known from Japanese Patent Laid-Open No. 130945/1982).

It is the object of the present invention to provide a process for easily producing a triaryl methacrylate in high purity by using an easily available starting material. Said object is achieved by a process which is characterized by reacting methacrylic acid with a triarylmethylating agent of formula II

$$Z^3CX \qquad (II)$$

wherein

X is halogen

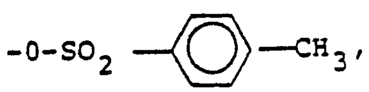, $-O-SO_2-CH_2$ - $C\ell O_4$, -CN and -SCN.

and

Z is as defined above,

in an inert, organic solvent containing a trialkylamine or an amine having a similar basicity in an amount of 1.5 to 10 mol per mol methacrylic acid or in the presence of an anion exchanger.

In the process of the present invention, side reactions hardly take place, the obtained products can be easily separated and pure esters can be obtained by merely recrystallizing the crude products and therefore the process of the present invention does not require purifying treatments by, for example, chromatography which sometimes causes hydrolysis.

It is preferred that Z in formula I is an unsubstituted or substituted phenyl group.

In one embodiment the process of the present invention is conducted in an inert organic solvent containing a trialkylamine or an amine having a similar basicity in an amount of 1.5 to 10 mol per mol methacrylic acid, precipitated amine salt is removed and the solvent and the excess amine are evaporated from the resulting solution. In a second embodiment the reaction is carried out in the presence of an anion exchanger. Each of these embodiments of the invention will be illustrated below.

First Embodiment of the Invention

In the present invention, methacrylic acid per se is used as the carboxylic acid component for producing esters and is reacted with a compound having a triphenylmethyl structure, such as triphenylmethyl bromide or chloride.

The polymerizable carboxylic acid is condensed with an easily hydrolyzable triphenylmethyl compound by using an amine instead of a silver salt according to the prior art. This reaction is carried out in an inert organic solvent to avoid hydrolysis.

A volatile amine such as triethylamine is used in an excess amount of 1.5 to 10 mol per mol of methacrylic acid.

The excess amine accelerates the condensation reaction and at the same time prevents the reaction system from being shifted to an acidic pH region. Though the reaction is carried out while inhibiting the incorporation of water in the reaction system, a trace amount of water is inevitably incorporated therein so that a prolonged reaction time and particularly a shift of the pH to an acidic side are deleterious, since the final product of the present invention undergoes hydrolysis. When the reaction is carried out while paying attention to the inhibition of the above-mentioned phenomenon, an amine salt such as triethylamine hydrobromide is precipitated in a relatively short time, for example, within two or three hours and the reaction is terminated. The obtained product can be directly crystallized by removing the amine salt by filtration and evaporating the solvent and an excess amine from the resulting reaction solution. If desired, the purity can be further improved by conventional techniques such as recrystallization.

The above-described simple method of obtaining the final products in a high purity has been realized because:

(1) triphenylmethyl methacrylate per se has good crystallizability;

(2) the reaction is carried out in the presence of an excess amine so that side reactions such as hydrolysis can be prevented; and

(3) the starting materials are chosen such that the amine salt and excess amine coexist with the product when the reaction is terminated and can be removed by simple means such as filtration and evaporation.

When acetic or acrylic acid is used instead of methacrylic acid in the process of the present invention, triphenylmethyl esters can be produced but they are not as pure nor directly crystallizable as the corresponding methacrylate. When benzoic acid is used, the reaction rate is slow as compared with methacrylic acid, and even when the reaction time is prolonged, large crystals cannot be obtained as in the case of the methacrylate.

TLC analysis reveals that triphenylmethyl alcohol and benzoic acid, both having high crystallizability, are incorporated.

The reaction of the present invention can be represented by the following equation:

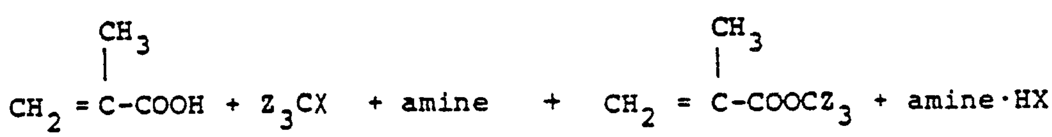

wherein Z is an unsubstituted or substituted phenyl group, and the three Z groups may be the same or different. The Z group can be represented by the following formula:

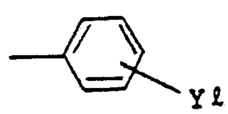

wherein Y is an alkyl group having 1 to 20 carbon atoms, an alkoxy group having 1 to 20 carbon atoms or a halogen atom (F, Cl, Br or I), and l is an integer of 0 to 5. Among these substituents, p-chloro and p-fluoro groups can be mentioned as typical halogen substituent. Preferred alkyl groups include $C_1$—$C_{10}$ alkyl groups, particularly m-methyl, 3,5-dimethyl and m-ethyl groups. Preferred alkoxy groups include $C_1$—$C_{10}$ alkoxy groups, particularly m-methoxy, 3,5-dimethoxy and m-ethoxy groups.

X is halogen,

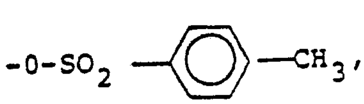, $-O-SO_2-CH_3$ - $ClO_4$, -CN and -SCN.

Typical compounds of the formula $Z_3CX$ include triphenylmethyl chlorides and triphenylmethyl bromides, but triphenylmethyl derivatives having substituted phenyl groups as exemplified above can be similarly used in the process of the present invention.

The reaction is carried out in an inert organic solvent. Preferably a solvent which does not dissolve the

formed amine salt is used. The solvent must be anhydrous, since otherwise the resulting ester is liable to undergo hydrolysis. Examples of typical solvents include paraffin hydrocarbons such as n-pentane and n-hexane; aromatic hydrocarbons such as benzene and toluene; halogenated hydrocarbons such as dichloromethane and carbon tetrachloride; acetonitrile and ethers such as diethyl ether and tetrahydrofuran (THF).

The amines used in the present invention include trialkylamines or amines having a similar basicity which are inert to the starting materials. Primary and secondary amines may be used unless side reactions such as a N-triphenylmethylation reaction take place under reaction conditions. It is desirable that the amine salt formed by the reaction is easily precipitated and can be filtered off from the organic solvent.

It is necessary that the amine is used in excess of methacrylic acid

If the amine is used in an amount of 1.5 to 10 mol per mol methacrylic acid, the reaction can be terminated in a short time and the reaction system can be prevented from being shifted to an acidic pH region.

A volatile amine is preferably used, because excess amine must be removed together with the solvent by evaporation after the removal of the amine salt by filtration.

Triethylamine is particularly preferred, because it is easy to handle as a liquid, can be easily removed by evaporation, is inert to the starting material and has an appropriate basicity. Alkylamines having 12 or less carbon atoms in total such as trimethylamine, tributylamine or diisopropylamine, may also be used, but have advantages and disadvantages with respect to volatility.

Even when a weak base such as pyridine is used, the amine can be precipitated from the reaction solution. Since, however, pyridine hydrobromide is acidic, the final ester is liable to be hydrolyzed by trace amounts of water contained in the solution. Therefore, trialkyl-amines or amines having a similar basicity are used.

The reaction can be carried out at about room temperature or while cooling with ice. If desired, the reaction can be carried out over a wide temperature range of, for example, −80 to 100°C, unless any problems of side reactions such as polymerization are caused. Cooling (for example, to 10 to 20°C) is often effective for accelerating the precipitation of the amine salt, and can be completed within 6 hours, for example, after 2 to 3 hours.

The precipitated amine salt can be easily removed by conventional solid-liquid separation methods such as filtration, and the solvent and excess amine are evaporated from the mother liquor. If necessary, the removal of the solvent and excess amine is carried out under reduced pressure. Triphenylmethyl methacrylate can be obtained directly as a crystal. If desired, the purity can be further improved by conventional techniques such as recrystallization.

In this way, final products in a crystalline form can be obtained from methacrylic acid in a short time and the formed ester is recovered by simple separation means such as filtration and evaporation.

Second Embodiment of the Invention

Commercially available methacrylic acid containing a polymerization inhibitor can be used as such.

The triarylmethylating agent is represented by the formula $Z_3CX$ wherein X is as defined above in the first embodiment of the invention and Z is an aryl group such as phenyl, α-naphthyl or β-naphthyl, which may be substituted with 1 to 5 groups, such as halogen (F, Cl, Br, or I), alkyl, alkoxy or alkenyl, which are inert to the reaction of the present invention. Particularly useful Z groups include a phenyl group and substituted phenyl groups having substituent Yl represented by the formula:

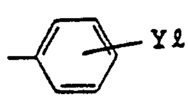

wherein l is an integer of 1 to 5 and Y is a halogen atom such as p-chloro or p-fluoro, an alkyl group having 1 to 10 carbon atoms such as m-methyl, 3,5-dimethyl or m-ethyl, or an alkoxy group having 1 to 10 carbon atoms such as m-methoxy, 3,5-dimethoxy or m-ethoxy. The three Z groups of the triarylmethyl group, $Z_3C$, may be the same (for example, triphenylmethyl) or different (for example, p-chlorophenyldiphenylmethyl).

The process of the present invention is characterized in that the reaction of methacrylic acid with the triarylmethylating agent is carried out in the presence of an anion exchange resin. The anion exchange resin includes a quaternary ammonium type of a strongly basic anion exchange resin and a tertiary amine type of a weakly basic anion exchange resins. The latter is preferred in the present invention. When the quaternary ammonium type of resin is used, there is the possibility that hydrolysis takes place due to water formed from the hydroxide anion and that polymerization of the starting material and the product takes place due to its strong basicity. When the tertiary amine type of resin is used, the active group is a weakly basic group such as a tertiary alkylamino or pyridyl group so that no hydrolysis or polymerization as mentioned above occur and the final ester can be formed by the condensation reaction between the starting materials at an appropriate reaction rate.

Examples of the tertiary amine type of anion exchange resins include commercially available resins such as Amberlite® IRA45, IRA47, IRA68, IRA93 and IRA94; Amberlyst® A—21 and A—26; Diaion® WA—10,

WA—11, WA—20, WA—21, WA—30, CR—20 and CR—40; and Dowex® MWA and WGR.

It is desirable to use an anion exchange resin which is insoluble in an organic solvent used for the reaction in order that the resin can be readily separated from the reaction mixture or repeatedly used.

A swelling of the anion exchange resin by the solvent is desirable, because soluble impurities are washed off from the resin, the reactivity is increased and the reaction proceeds with good selectivity in a short time to give the final product as a crystal.

Methacrylic acid may be reacted with the triarylmethylating agent in an equivalent ratio. However, it is preferred that methacrylic acid is used in excess of the triarylmethylating agent, for example, in a quantity of 1.5 times by equivalent that of the triarylmethylating agent to completely consume the triarylmethylating agent by the reaction and to prevent by-products such as triarylmethyl alcohol from being formed. Usually, methacrylic acid is used in a quantity of 1.0 to 1.1 times by equivalent that of the triarylmethylating agent.

The reaction is carried out in an inert organic solvent. The solvent is chosen by taking the solubility of the reactants and the action of the solvent on the anion exchange resin into consideration. For example, when Amberlyst® A—21 is used as the anion exchange resin, methylene chloride is preferred as solvent, since it can swell the resin but not dissolve it and can dissolve the triarylmethylating agent. This solvent scarcely dissolves water and commercially available methylene chloride contains only a little water so that it is not necessary to dry the methylene chloride solvent prior to the reaction when the reaction can be terminated in a relatively short time. Other solvents can be chosen from inert solvents such as acetonitrile or ethyl ether, according to the kinds of the anion exchange resins. However, a solvent which can incorporate water dissolved therein such as ethyl ether, is sometimes not preferred, since when the solvent is used for the reaction over a long period of time without preliminary dehydration treatment, there is a possibility that triphenylmethyl alcohol is formed as a by-product.

The simplest reaction mode includes a flow method which comprises passing a solution containing both starting materials through an anion exchange resin layer. Alternatively, a solution of methacrylic acid is first fed to a resin layer to allow the acid to be supported on the resin layer and then the triphenylmethylating agent is passed therethrough to effect the reaction.

Thereby, an ester is formed and flows out, leaving an acid, HX, formed during condensation. The reaction time can be controlled by the quantity of the packed resin and the flow velocity of the solution.

The reaction may be carried out batchwise. For example, the solution containing the resin dispersed therein is reacted under stirring. This method often gives results similar to those obtained by the flow method.

The reaction can be carried out at about room temperature, for example, at a temperature of 0 to 50°C and can be completed within several minutes. If desired, the reaction may be carried out over a wide temperature range of −80 to 100°C unless any problems of side reactions such as polymerization are caused.

It is necessary that the anion exchange resin has an ion exchange capacity of at least 1 mol per mol of the substrate, and it is preferred to use the resin having an ion exchange capacity of 1 to 20-fold mol, particularly 5 to 10-fold mol.

By carrying out the reaction in the presence of the anion exchange resin, methacrylic acid is rapidly condensed with the triarylmethylating agent in the solution to form a triarylmethyl methacrylate.

A hydroacid HX (for example, hydrohalogenic acid, p-toluenesulfonic acid or perchloric acid) of a group thereby eliminated in the reaction forms a salt with the anion exchange resin so that the hydrolysis of the produced ester can be minimized even in the presence of some water in the system. Since the reaction solution separated from the solid resin does not contain any salts formed by the condensation, crude crystal of the purpose triarylmethyl methacrylate can be obtained in a high yield merely by distilling off the solvent.

Thus, the process of the present invention hardly suffers from side reactions and the salt formed by the condensation can be separated in the form of a resin salt from the reaction solution so that the ester can be easily obtained in pure form by crystallization. Accordingly the process of the present invention does not require purifying treatments by, for example, chromatography which sometimes causes hydrolysis.

The anion exchange resin used as a condensing agent can be easily regenerated by washing it with an alkali and can be repeatedly used. Thus, the present invention is also in this respect superior to the prior art processes where a metal salt or a low-molecular amine is used.

It has been found that the process of the present invention can be accomplished only by a specific combination wherein methacrylic acid is used as a carboxylic acid component and the triarylmethyl group is used as a tertiary alcohol component. When an acyclic compound such as t-butyl bromide or chloride is used as the tertiary alcohol component, the compound does not react with methacrylic acid. When acrylic or acetic acid is used as the carboxylic acid component, only a trace quantity of the triphenylmethyl ester is formed. Accordingly, it is an unexpected finding that methacrylic acid is rapidly condensed with the triarylmethylating agent in the presence of an anion exchange resin and the final product can be obtained in the form of crystals only by distilling off the solvent from the reaction solution.

The following examples further illustrate the present invention.

The NMR and IR data in the examples were determined by the following methods. The $^1H$ NMR spectrum was measured using TMS as internal reference by JEOL JNM—NH—100. The IR spectrum was measured by JASCO IRA—2.

Example 1

5.05 g (0.05 mol) of dry triethylamine distilled in the presence of calcium hydride was added to 20 ml of anhydrous ether distilled in the presence of lithium aluminum hydride, and the mixture was cooled with ice in a nitrogen atmosphere. 0.86 g (0.01 mol) of methacrylic acid was added thereto and the mixture was stirred. While cooling the mixture with ice, 3.23 g (0.01 mol) of triphenylmethyl bromide dissolved in 10 ml of anhydrous ether was added thereto, whereby the reaction rapidly proceeded and triethylamine hydrobromide was precipitated. The reaction was continued for 2.5 h until the salt was completely precipitated, and the resulting crystals were recovered by filtration. When the solvent and excess triethylamine were distilled off, triphenylmethyl methacrylate was obtained in crystalline form, which was then recrystallized from ether to give 3.07 g (yield: 92.9%) of colorless prismatic crystals.

m.p.: 100—101°C (Lit.: 101—102°C);
IR (C $Cl_4$): 1725, 1490, 1140 $cm^{-1}$;
$^1$HNMR ($CDCl_3$): δ 1.99 (3H, dd, J=1.0, 1.4$H_Z$), 5.60, (1H, m), 6.23 (1H, m), 7.2—7.5 (15H, m);
MS: m/z 328 ($M^+$).

Example 2

The procedure of Example 1 was repeated except that the reaction was carried out in dry THF at room temperature. 3.20 g (yield: 96.8%) of triphenylmethyl methacrylate was obtained.

Example 3

The procedure of Example 2 was repeated except that 2.79 g (0.01 mol) of triphenylmethyl chloride was used. 3.03 g (yield: 91.7%) of triphenylmethyl methacrylate was obtained.

Example 4

The procedure of Example 2 was repeated except when 3.96 g (0.05 mol) of dry pyridine was used instead of triethylamine and the reaction was carried out in THF.

When the reaction solution was examined by means of thin-layer chromatography it was confirmed that triphenylmethyl methacrylate was quantitatively formed. After the amine salt was removed by filtration, the solvent and excess pyridine were evaporated. The final product was obtained as syrup. An attempt to recrystallize it from ether and n-hexane failed, since hydrolysis took place due to water contained in the solvents for recrystallization and triphenylmethyl alcohol was formed.

Example 5

The procedure of Example 4 was repeated except that 2.79 g of triphenylmethyl chloride was used. The final product was quantitatively formed and separated as syrup, which was then recrystallized from a thoroughly dried solvent. Crystals similar to those of Example 1 were obtained.

Comparison Example 1

For comparison, 1.01 g (equivalent) of triethylamine was used and an experiment similar to that described in Example 1 was carried out. The concentrate obtained by filtering the amine salt and evaporating the solvent contained triphenylmethyl alcohol, and the final product having a high purity similar to that of the product of Example 1 could not be obtained.

Example 6

10 g of Amberlyst® A—21 as a weakly basic anion exchange resin containing 0.0047—0.005 g of the active group per g of the resin, on a dry basis, was weighed and washed with methanol and acetone to remove water.

The resin was then dispersed in 20 ml of methylene chloride to swell it, and then 0.86 g (0.01 mol) of methacrylic acid was added thereto. The mixture was stirred at room temperature. 3.23 g (0.01 mol) of triphenylmethyl bromide dissolved in 20 ml of methylene chloride was added thereto. The reaction was terminated within 10 minutes.

The resin was removed by filtration and the solvent was distilled off from the reaction solution, whereby the final product was obtained as crude crystals. The product was recrystallized from ether to give 3.12 g (yield: 94.5%) of triphenylmethyl methacrylate as colorless prismatic crystal

M.P.: 100 ~ 101°C (Lit. 101 ~ 102°C)
IR($CCl_4$): 1725, 1490, 1140 $cm^{-1}$
$^1$H NMR ($CDCl_3$): δ 1.99 (3H, dd, J=1.0, 1.4 Hz), 5.60 (1H, m), 6.23 (1H, m), 7.2 ~ 7.5 (15H, m)
MS: m/z 328($M^+$)

Example 7

The procedure of Example 6 was repeated except that 2.37 g (0.0085 mol) of triphenylmethyl chloride was used instead of triphenylmethyl bromide. 2.26 g (yield: 80.4%) of triphenylmethyl methacrylate was obtained.

Example 8

The same starting materials as those of Example 7 were used and the reaction was carried out in ether (not previously dehydrated) as solvent. The resin was immersed in ether overnight, but did not swell unlike the immersion in methylene chloride and the reaction required a long time.

Ether was evaporated from the reaction solution to obtain crystals of triphenylmethyl alcohol (yield: 50.9%). Triphenylmethyl methacrylate (yield: 28.7%) was obtained from the mother liquor.

Example 9

A glass column of 1.5 cm in diameter and 25 cm in height was packed with 20 g of Amberlyst® A—21 and the packed layer was wetted with 20 ml of methylene chloride. A solution of a substrate composed of 0.86 g (0.01 mol) of methacrylic acid and 3.23 g (0.01 mol) of triphenylmethyl bromide dissolved in 20 ml of methylene chloride was passed through the layer downward over a period of 30 min.

Methylene chloride was distilled off from the effluent to obtain 2.90 g (yield: 87.9%) of triphenylmethyl methacrylate as crude crystals. The product was recrystallized from ether to give 2.79 g (yield: 84.4%) of pure crystals.

Example 10

The procedure of the flow method of Example 9 was repeated except that 2.79 g (0.01 mol) of triphenylmethyl chloride was used instead of triphenylmethyl bromide. After recrystallization, 2.84 g (yield: 86.1%) of triphenylmethyl methacrylate was obtained.

Example 11

The anion exchange resin used in Example 6 was regenerated by washing with 500 ml of a 4% aqueous caustic soda solution and then with methanol and acetonitrile to remove water. 0.43 g (0.005 mol) of methacrylic acid and 1.39 g (0.005 mol) of triphenylmethyl chloride were dissolved in methylene chloride and reacted together batchwise in the presence of the above regenerated resin to give 1.59 g (yield: 96.0%) of triphenylmethyl methacrylate.

Comparison Example 2

The procedure of each of Examples 6 and 7 was repeated except that 0.01 mol of acrylic acid was used instead of methacrylic acid. Only a trace quantity of triphenylmethyl acrylate was formed.

The procedure of Example 6 was repeated except that 0.01 mol of acetic acid was used. Only a trace quantity of triphenylmethyl acetate was formed.

Comparison Example 3

The procedure of Example 6 was repeated except that 0.01 mol of t-butyl bromide was used instead of triphenylmethyl bromide. No reaction took place. t-Butyl chloride gave similar results.

**Claims**

1. A process for producing a triarylmethyl methacrylate of formula I

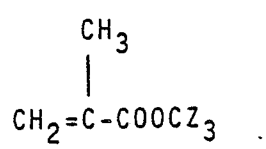

(I)

wherein Z is an aryl group which may be substituted, characterized by reacting methacrylic acid with a triarylmethylating agent of formula II

$$Z_3CX \quad \text{(II)}$$

wherein

X is halogen,

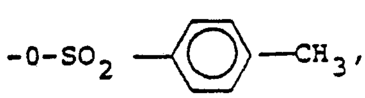, $-O-SO_2-CH_2$ - $C\ell O_4$, -CN and -SCN.

and

Z is as defined above,

in an inert, organic solvent containing a trialkylamine or an amine having a similar basicity in an amount of 1.5 to 10 mol of methacrylic acid, or in the presence of an anion exchanger.

2. The process of claim 1 characterized in that Z is an unsubstituted or substituted phenyl group.

3. The process of claim 1 or 2, characterized in that the organic solvent does not dissolve the amine salt

produced in the reaction and is substantially free from water.

4. The process of any of claims 1 to 3 characterized in that the reaction is conducted at a temperature of from −80 to 100°C.

5. The process of claim 1, characterized in that said anion exchanger is a quaternary ammonium type of a strongly basic anion exchange resin or a tertiary amine type of a weakly basic anion exchange resin.

**Patentansprüche**

1. Verfahren zur Herstellung eines Triarylmethylmethacrylats der Formel I

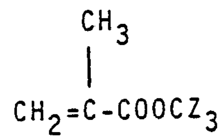 (I)

worin Z eine Arylgruppe ist die substituiert sein kann, dadurch gekennzeichnet, daß Methacrylsäure mit einem Triarylmethylierungsmittel der Formel II

$$Z_3CX \quad (II)$$

worin

X Halogen,

$-O-SO_2$ 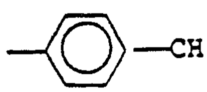 $-CH_3$, $-O-SO_2-CH_2$ - $ClO_4$, $-CN$ oder $-SCN$.

ist und

Z die vorstehend angegebene Bedeutung besitzt,

in einem inerten, organischen Lösungsmittel, das ein Trialkylamin oder ein Amin mit einer ähnlichen Basizität in einer Menge von 1,5 bis 10 Mol pro Mol Methacrylsäure enthält, oder in Gegenwart eines Anionenaustauschers umgesetzt wird.

2. Verfahren nach Anspruch 1, dadurch gekennzeichnet, daß Z eine unsubstituierte oder substituierte Phenylgruppe ist.

3. Verfahren nach Anspruch 1 oder 2, dadurch gekennzeichnet, daß das organische Lösungsmittel das Aminsalz, das in der Reaktion gebildet wird, nicht auflöst und im wesentlichen wasserfrei ist.

4. Verfahren nach einem der Ansprüche 1 bis 3, dadurch gekennzeichnet, daß die Reaktion bei einer Temperatur von −80 bis 100°C durchgeführt wird.

5. Verfahren nach Anspruch 1, dadurch gekennzeichnet, daß der Anionenaustauscher ein stark basisches Anionenaustauschharz vom quaternären Ammonium typ oder ein schwach basisches Anionenaustauschharz vom tertiären Amintyp ist.

**Revendications**

1. Procédé pour la préparation d'un méthacrylate de triarylméthyle de formule I

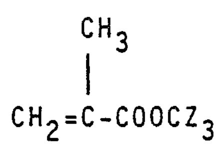 (I)

dans laquelle Z est un groupe aryle éventuellement substitué, caractérisé par la réaction de l'acide méthacrylique avec un agent de triarylméthylation, de formule II

$$Z_3CX \quad (II)$$

dans laquelle

X est un atome d'halogène ou un groupe

$-O-SO_2$ 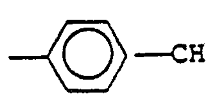 $-CH_3$, $-O-SO_2-CH_2$ - $ClO_4$, $-CN$ ou $-SCN$.

et

Z et tel que défini plus haut,

dans un solvant organique inerte, contenant une trialkylamine ou une amine ayant une basicité semblable, en une quantité de 1,5 à 10 moles par mole d'acide méthacrylique, ou en présence d'un échangeur d'anions.

2. Procédé selon la revendication 1, caractérisé en ce que Z est un groupe phényle substitué ou non substitue.

3. Procédé selon la revendication 1 ou 2, caractérisé en ce que le solvant organique ne dissout pas le sel d'amine produit dans la réaction et est pratiquement exempt d'eau.

4. Procédé selon l'une quelconque des revendications 1 à 3, caractérisé en ce que l'on effectue la réaction à une température de −80 à +100°C.

5. Procédé selon la revendication 1, caractérisé en ce que ledit échangeur d'anions est un type ammonium quaternaire d'une résine échangeuse d'anions, fortement basique, ou un type amine tertiaire d'une résine échangeuse d'anions, faiblement basique.